# EUROPÄISCHE PATENTSCHRIFT

(11) **EP 0 502 962 B1**
(45) Veröffentlichungstag und Bekanntmachung des Hinweises auf die Patenterteilung: **13.03.1996**
(21) Anmeldenummer: 91900751.8
(22) Anmeldetag: 22.11.1990
(51) Int. Cl.: C07K 14/815, C07K 17/08, A61K 38/58, A61K 47/48

(54) **Hirudin-Muteine und deren Polyalkylenglykolkonjugate**
Hirudinmuteins and their polyalkylene glycol conjugates
Mutéines de l'hirudine et leurs conjugues de polyalkyleneglycol

(30) Priorität: 01.12.1989 DE 3939800
(43) Veröffentlichungstag der Anmeldung: 16.09.1992
(73) Patentinhaber: BASF Aktiengesellschaft, D-67063 Ludwigshafen (DE)
(72) Erfinder: KURFUERST, Manfred, D-6733 Hassloch (DE); RUEBSAMEN, Klaus, D-6730 Neustadt (DE); SCHMIED, Bernhard, D-6710 Frankenthal (DE); KOERWER, Wolfgang, D-6718 Gruenstadt (DE); SCHWEDEN, Jürgen, D-6705 Deidesheim (DE); HOEFFKEN, Hans, Wolfgang, D-6700 Ludwigshafen (DE)
(86) Internationale Anmeldenummer: EP9001998
(87) Internationale Veröffentlichungsnummer: WO9108229

(56) Entgegenhaltungen:
- EP-A- 0 236 987
- EP-A- 0 324 712
- EP-A- 0 332 523
- EP-A- 0 341 215
- EP-A- 0 345 616
- EP-A- 0 367 489

## Beschreibung

Die Erfindung betrifft neue Hirudinmuteine und deren Polyalkylenglykolkonjugate, deren Herstellung sowie deren Verwendung sowohl zur Prophylaxe und Therapie von kardiovaskulären Krankheiten als auch zur Modifikation makromolekularer Träger.

Hirudin ist ein seit langer Zeit bekanntes, natürlich vorkommendes Protein mit blutgerinnungshemmenden Eigenschaften. Es ist der stärkste und selektivste bisher bekannte Thrombininhibitor (Naturwissenschaften, (1955) 42, 537; Hoppe-Seylers Z. für Biol. Chemie, (1985) 366, 379). Das aus dem medizinischen Blutegel Hirudo medicinalis isolierbare Polypeptid besteht aus 65 Aminosäuren, enthält drei Disulfidbrücken und ist an Position Tyrosin 63 sulfatiert. Darüber hinaus existieren noch mehrere natürlich vorkommende Isoformen, die sich vom ursprünglichen Hirudin durch Aminosäureaustausch in verschiedenen Positionen unterscheiden (Folia Haematol. (1988), 115, 30). Ebenso sind gentechnologisch hergestellte Varianten bekannt (Biochemistry (1988), 27, 6517, FEBS-Lett. (1988), 229, 87). Hirudin und verschiedene Varianten sind heute auf gentechnologischem Weg zugänglich, wobei den mit gentechnologischen Methoden hergestellten Hirudinen der Sulfatrest an Aminosäure Tyr 63 fehlt (Biochemistry (1989), 28, 2941, DNA (1986), 5, 511). Die gute physiologische Verträglichkeit dieses Gerinnungsinhibitors ist ebenfalls seit geraumer Zeit bekannt (Pharmazie (1981), 10, 653).

Trotz seiner günstigen pharmakodynamischen Eigenschaften ist Hirudin aufgrund seiner geringen Halbwertzeit im Blut von ca. 50 min für lang andauernde therapeutische Anwendungen weniger geeignet. Es ist bekannt, daß die Halbwertszeit von Proteinen durch Konjugation mit Makromolekülen verlängert werden kann (J. Biol. Chem. (1977), 252, 3582; Biochim. Biophys. Acta (1981), 660, 293). Häufig wird nach einer solchen Derivatisierung mit z.B. Polyethylenglykol eine signifikante Verschlechterung der enzymatischen Aktivität beobachtet, was die Anwendbarkeit solcher modifizierter Proteine stark einschränkt (Cancer. Treat. Rep. (1979), 63, 1127; Chemistry Lett. (1980), 773). Im Falle von Hirudin konnte von Walsmann kürzlich gezeigt werden, daß durch Kopplung mit Dextran eine deutliche Halbwertszeitverlängerung von ca. 50 min auf über 7 h erreicht werden konnte, allerdings unter drastischem Verlust an Aktivität (Pharmazie (1989), 44, 72). Einer therapeutischen Anwendung solcher Dextan-Hirudine steht trotz der günstig veränderten Halbwertszeit die sehr geringe Ausbeute an Produkt, die drastisch reduzierte spezifische Aktivität sowie die damit möglicherweise verbundenen Änderungen der pharmakodynamischen Eigenschaften entgegen.

Eine Konjugation von Proteinen mit Makromolekülen wird häufig über die Reaktion der Carboxylgruppen der Aminosäuren Asparaginsäure oder Glutaminsäure, über die Reaktion der Sulfhydrylgruppe der Aminosäure Cystein oder durch Umsetzung der Seitenketten-Aminogruppe der Aminosäure Lysin des entsprechenden Proteins erreicht. Häufig sind jedoch gerade die genannten Aminosäuren für die Funktion des entsprechenden Proteins von essentieller Bedeutung. Die Derivatisierung eines Proteins kann mit einer Veränderung der physikalisch/chemischen oder enzymatischen Eigenschaften bis hin zur Inaktivierung verbunden sein. Hirudin enthält mehrere Asparaginsäure- und Glutaminsäurereste, hauptsächlich im C-terminalen Bereich des Moleküls. Lysinreste befinden sich in Position 27, 36 und 47 im Hirudinmolekül. Darüber hinaus wäre eine Kopplung über den C-Terminus oder den N-Terminus des Moleküls denkbar. Es ist jedoch bekannt, daß sowohl die sauren Aminosäuren im C-terminalen Bereich (FEBS. Lett. (1983), 164, 307-313) als auch die basischen Lysinreste, insbesondere der im Molekül stark exponierte Lysinrest Nr. 47 an der Interaktion des Hirudins mit der Protease Thrombin entscheidend beteiligt sind (Biol. Chem. Hoppe-Seyler (1985), 366, 379-385). Reaktionen am N-Terminus, wie z.B. eine Verlängerung (Biochemistry (1989), 28, 10079) führen zu einer drastischen Abnahme der inhibitorischen Aktivität des Hirudins. Daher war nicht zu erwarten, daß eine Derivatisierung von Hirudin ohne signifikanten Aktivitätsverlust zu erreichen ist. Diese Erwartung wird durch die von Walsmann (Pharmazie (1989), 44, 72) durchgeführten Arbeiten zur Derivatisierung der Lysinreste des Hirudins mit Dextran deutlich belegt.

Wegen der großen Zahl saurer Aminosäuren ist bei einer Konjugation von Makromolekülen mit den Carboxylseitenketten des Hirudins kein einheitliches Produkt zu erwarten. Selbst bei einer Derivatisierung nur der basischen Funktionen des Polypeptids wird ein Gemisch aus bis zu 32 verschiedenen Verbindungen erwartet. Bei den mono-, di- und trisubstituierten Derivaten ist eine große Zahl von Stellungsisomeren denkbar, die sich in ihren physikalischen und chemischen Eigenschaften weitgehend gleichen, jedoch in ihrer biologischen Aktivität unterschiedlich sind. Selbst wenn die Mehrzahl der theoretisch denkbaren Konjugate nur im Spurenbereich zur Gesamtmischung beitragen, muß mit erheblichen Problemen bei der Trennung von einem inhomogenen Produkt gerechnet werden.

Es wurde nun gefunden, daß die Konjugation von Polyalkylenglykolderivaten durch Einsatz geeigneter Hirudin-Muteine so gut zu steuern ist, daß mit akzeptablem Reinigungsaufwand chemisch einheitliche Hirudin-Polymerderivate erhalten werden können. Überraschend war der Befund, daß Konjugate der Formel I aus Polyalkylenglykol oder Polyalkylenglykolderivaten mit blutgerinnungshemmenden Hirudin-Muteinen

[z-(CH₂)ₙ-[O-(CH₂)ₙ]ₘ-W-]ₚHir I

worin
- z: einen der Reste -OH, -NH₂, -NH-CO-R, -O-R oder -O-CO-R (mit R in der Bedeutung einer C₁-C₄-Alkylgruppe),
- n: die Zahl 2, 3 oder 4,
- m: eine Zahl von 50 bis 500,
- W: eine direkte kovalente Bindung oder einen Linker,
- p: die zahl 1, 2 oder 3
bedeuten und
Hir für ein über die Aminogruppe(n) der Lysinseitenkette(n) an den (die) z-(CH₂)ₙ-[O-(CH₂)ₙ]ₘ-W-Rest(e) gebundenes Polypeptid der Formel II 
A -Tyr-Thr-Asp-Cys-Thr-Glu-Ser-Gly-
10
Gln-Asn-Leu-Cys-Leu-Cys-Glu-Gly-Ser-Asn-
20
Val-Cys-Gly- B -Gly-Asn- C -Cys-Ile-Leu-
30 II
Gly-Ser-Asp-Gly-Glu-Lys-Asn-Gln-Cys-Val-
40
Thr-Gly-Glu-Gly-Thr-Pro- D -Pro-Gln-Ser-
50
His-Asn-Asp-Gly-Asp-Phe-Glu- E -Ile-Pro-
60
Glu-Glu-Tyr-Leu- F
65
 steht, mit der Bedeutung von
- A: = Val-Val,
= Ile-Thr,
= Leu-Thr oder
= Pro-Val,
- B: = Gln oder Glu,
- C: = Lys, Arg oder Asn,
- D: = Lys, Arg, Asn oder Gln,
- E: = Glu oder Pro,
- F: = Asp oder Gln,
wobei im Polypeptid II ein oder mehrere Aminosäuren in den Positionen 30-38 ausgetauscht oder deletiert und ein oder zwei zusätzliche Lysinreste durch Substitution einer vorhandenen Aminosäure in den Positionen 30 bis 38 enthalten sind, deutlich verlängerte Bioverfügbarkeiten besitzen, wobei die biologische Wirksamkeit ganz oder weitgehend erhalten bleibt.

Die Erfindung betrifft weiter die Polypeptide der Formel II.

Im Interesse einer einfacheren Reaktionsführung und eines chemisch einheitlichen Produktes ist es von Vorteil, Lysinreste bei denen eine Umsetzung mit PEG nicht gewünscht wird, durch andere, schwächer nucleophile Aminosäuren zu ersetzen. Als besonders günstig hat bei den obengenannten Muteinen der Austausch von Lys 47 gegen Arg 47 oder auch Gln 47 erwiesen. Die entsprechenden PEG-Mutein-Kopplungsprodukte besitzen ähnliche hohe spezifische Aktivitäten wie das underivatisierte Mutein. Durch die Anzahl der vorhandenen bzw. neu eingefügten Lysinreste kann auf den Anteil des koppelbaren Polymers und damit auf das Molgewicht des Konjugates Einfluß genommen werden.

Solche Hirudinmuteine lassen sich sehr gut auf gentechnologischem Weg herstellen. Zunächst stellt man die für das jeweilige Mutein kodierende Nukleinsäure synthetisch her. Diese synthetischen Gene lassen sich dann mit geeigneten Regulatorsequenzen (Promotor, Terminator etc.) versehen und in heterologen Systemen zur Expression bringen (FEBS-Lett. (1986) 202, 373 (1986); Biol. Chem. Hoppe-Seyler (1986) 367, 731). Die Expression kann in eukaryontischen Systemen (Mammaliazellen, Hefen oder filamentösen Pilzen) oder in prokaryontischen Systemen (E. coli, Bacilli etc.) erfolgen. Die Expression in E. coli erfolgt bevorzugt über ein Fusionsprotein, aus dem das Hirudin freigesetzt und anschließend aktiviert werden kann (DNA (1986) 5, 511). In den folgenden Beispielen wird exemplarisch die Herstellung von nur einigen der erfindungsmäßig beanspruchten Muteine beschrieben, die übrigen Muteine sind analog zugänglich.

Als Linker W kommen folgende Gruppen in Betracht:
-X-CO-; -X-CO-NH-U-NH-CO-; -X-CO-CH₂-CH₂-CO-; -X-CH₂-CO-oder 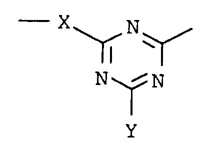
- X: in der Bedeutung von -S-, -O-, -NH- und
- U: in der Bedeutung einer C₂-C₆-Alkylengruppe oder einer p-Phenylengruppe und
- Y: in der Bedeutung von -Cl, -OH, oder H.

Die neuen Hirudinpolyalkylenglykolderivate lassen sich herstellen, indem man Hirudin-Muteine der Formel II umsetzt mit Polyalkylenglykolderivaten der Formel III

Z-(CH₂)ₙ-[O-(CH₂)ₙ]ₘ-E III

worin
- Z,: m und n die bereits angegebene Bedeutung besitzen und
- E: einen der Reste -X-CO-V, -X-CO-NH-U-N=C=O, -X-CO-CH₂-CH₂-CO-V, -X-CH₂-CO-V, 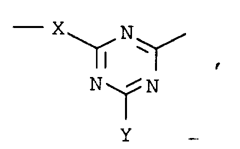 oder -O-SO₂-R mit X, U und Y in der angegebenen Bedeutung und
- V: in der Bedeutung von 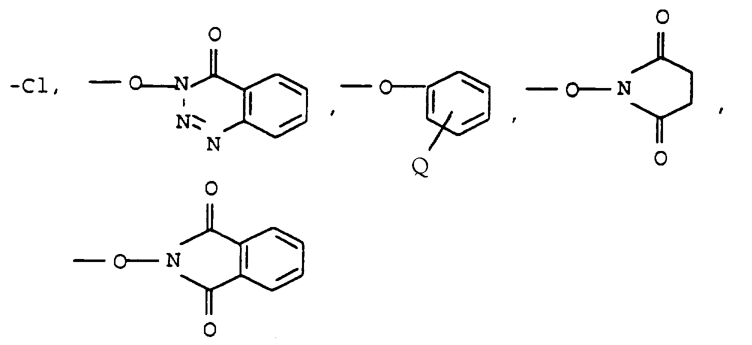
mit Q in der Bedeutung von 1-3 Halogenatomen oder 1-2 Nitrofunktionen oder einer Acetyl-Gruppe und
R in der Bedeutung von Methyl, Ethyl, n-Propyl, i-Propyl, Phenyl, Tolyl oder Tresyl.

Die Umsetzung von III mit Hirudin-Muteinen geschieht wie folgt:

Das aktivierte Polyalkylenglykol der Formel III wird in stöchiometrischen Mengen oder mit einem Überschuß in einem geeigneten Puffer (pH 6-10), in Wasser, gegebenenfalls unter Zusatz einer Hilfsbase wie Natrium- oder Kaliumcarbonat oder -hydrogencarbonat, Alkalihydroxid, Triethylamin, N-Methylmorpholin, Diisopropylamin oder Pyridin in einem organischen Lösungsmittel (Methanol, Ethanol, Isopropanol, Acetonitril, Dimethylformamid, N-Methylpyrrolidon, Dichlormethan, Dimethylsulfoxid, Tetrahydrofuran, 1,4-Dioxan, Toluol) oder in Gemischen der genannten Lösungsmittel bei Temperaturen zwischen -20°C und +100°C, bevorzugt bei Temperaturen zwischen 0°C und +60°C, mit dem Hirudin-Mutein zur Reaktion gebracht. Die entstandenen Konjugate werden isoliert und mit den in der Proteinchemie üblichen Methoden gereinigt und charakterisiert.

Die erfindungsgemäß beschriebenen Polyalkylen-Hirudin-Mutein-Konjugate zeigen ein im Vergleich zu Hirudin günstigeres pharmakologisches Wirkprofil. Sie besitzen nicht nur die vorteilhaften Pharmakodynamischen Eigenschaften des Hirudins, sondern weisen darüber hinaus eine erheblich verlängerte biologische Wirksamkeit und eine bessere Bioverfügbarkeit auf. Darüber hinaus zeigen Polyalkylglykol-Hirudin-Mutein-Konjugate im Vergleich zum Hirudin eine deutlich geringere Antigenität. Aufgrund dieser Eigenschaften sind die beschriebenen Polyalkylen-Konjugate Hirudin, Heparin und niedermolekularem Heparin bei der Therapie und Prophylaxe thromboembolischer Erkrankungen überlegen. Sie lassen sich zum Beispiel erfolgreich bei Myocardinfarkt, bei tiefer Venenthrombose, peripherer arterieller Verschlußkrankheit, Lungenembolie sowie bei extracorporaler Zirkulation z.B. Hämodialyse oder dem cardio-pulmonaren Bypass verwenden. Darüber hinaus können die Polyalkylenglykol-Hirudin-Mutein-Konjugate zur Verhinderung der Reocclusion nach Wiedereröffnung arterieller Gefäße durch mechanische Methoden oder Lyse verwendet werden. Weiterhin können die neuen Hirudin-Mutein-Polyalkylenglykol-Derivate erfolgreich zur Beschichtung von künstlichen Oberflächen, wie z.B. Hämodialysemembranen und den dazu erforderlichen Schlauchsystemen, bei Gefäßersatz oder bei Herz-Lungenmaschinen eingesetzt werden.

Die erfindungsgemäßen Verbindungen können in üblicher Weise oral oder parenteral (subkutan, intravenös, intramuskulär, intraperitoneal) verabfolgt werden.

Die Dosierung hängt vom Alter, Zustand und Gewicht des Patienten sowie von der Applikationsart ab. Je nach Applikationsform und Indikation beträgt die übliche Wirkstoffdosis in der Regel zwischen etwa 20 bis 40.000 ATU/kg Körpergewicht.

Die neuen Verbindungen können in den gebräuchlichen galenischen Applikationsformen fest oder flüssig angewendet werden, z.B. als Lösungen, Salben, Cremes oder Sprays. Diese werden in üblicher Weise hergestellt. Die Wirkstoffe können dabei mit den üblichen galenischen Hilfsmitteln wie Füllstoffen, Konservierungsmitteln, Fließreguliermitteln, Netzmitteln, Dispergiermitteln, Emulgatoren, Lösungsmitteln und/oder Treibgasen verarbeitet werden (vgl. H. Sucker et al: Pharmazeutische Technologie, Thieme-Verlag, Stuttgart, 1978).

### Beispiel 1

### Herstellung der Hirudin-Muteine

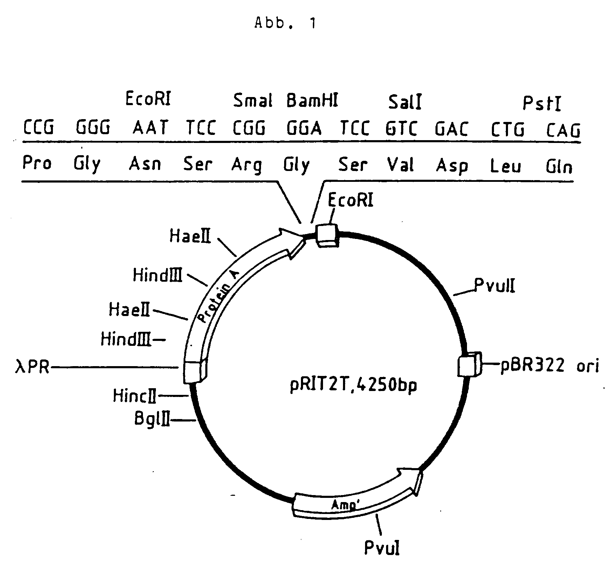

a) Konstruktion des Vektors
   Der Protein A-Vektor pRIT 2T (Abb. 1) ist kommerziell erhältlich und ausführlich beschrieben (Pharmacia Bestell Nr. 27-4808-01). Mit seiner Hilfe lassen sich Peptide und Proteine als Fusionsprotein mit Protein A aus Staphylococcus aureus in E. coli herstellen. Dazu muß die zu exprimierende Nukleinsäure unter Beibehaltung des Protein A-Leserasters in den Polylinker des Vektors pRIT 2T eingesetzt werden. Die pRIT 2T DNA wurde mit den Restriktionsendonukleasen Eco RI und Sal I nach Angaben der Hersteller geschnitten, der Spaltungsansatz über ein niedrigschmelzendes Agarosegel aufgetrennt und das größere Vektorfragment aus dem Gel in reiner Form isoliert. Die grundlegenden Techniken der Gentechnologie, sowie ausführliche Arbeitsanweisungen finden sich in Sambrook et al. (1989) "Molecular cloning" 2nd edition, CSH-Press.
b) Die für die erfindungsgemäß beanspruchten Hirudinmuteine kodierenden Sequenzen wurden mit Hilfe eines DNA-Synthesegerätes der Fa. Applied Biosystems, Modell 380A nach Vorschrift und mit den Chemikalien des Herstellers synthetisiert. Die komplette kodierende Region wurde dabei aus 3 Teilsequenzen zu je zwei komplementären Oligonukleotiden zusammengestellt. Die zueinander komplementären Oligonukleotide wurden zusammengegeben, 5 min auf 90°C erhitzt und über einen Zeitraum von 30 min auf Raumtemperatur abgekühlt. Die dabei entstehenden doppelsträngigen Fragmente wurden an ihren 5'-Enden kinasiert und zusammenligiert. Das so gebildete Hirudingen konnte in die Eco RI und Sal I Stelle des linearisierten Expressionsvektors pRIT 2T in der richtigen Orientierung und unter Beibehaltung des Protein A-Leserasters eingefügt werden.

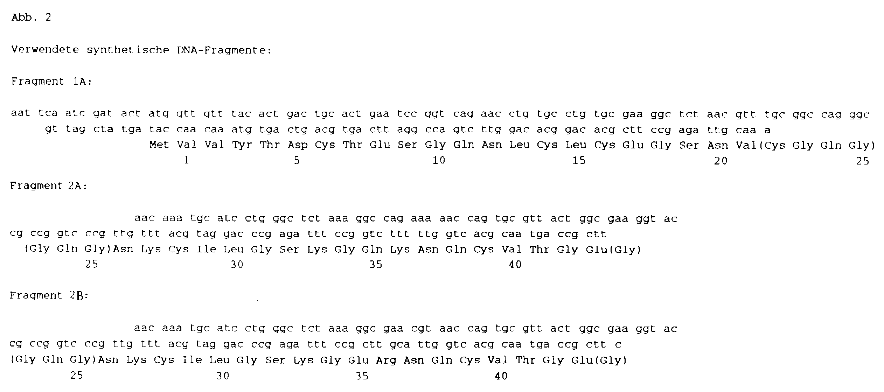

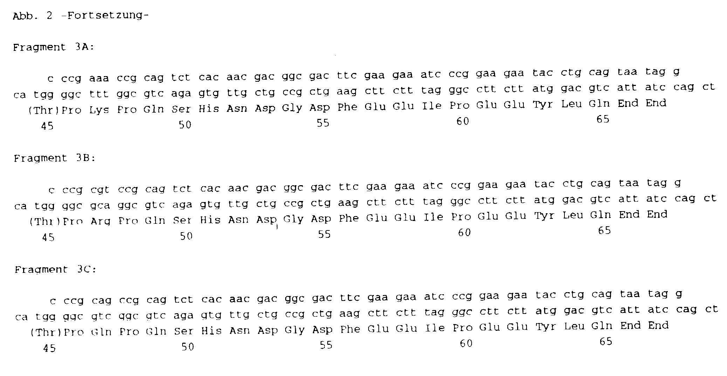

Zur Herstellung der verschiedenen Muteine wurden folgende Sequenzen (Abb. 2) miteinander kombiniert:

| | | |
|---|---|---|
| HL 11A | 1A+2A+3A | Ser32→Lys32 |
| | | Asp33→AsN33 |
| | | Glu35→GlN35 |
| HL 11B | 1A+2A+3B | Ser32→Lys32 |
| | | Asp33→AsN33 |
| | | Glu35→GlN35 |
| | | Lys47→Arg47 |
| HL 12A | 1A+2B+3A | Asp33→Lys33 |
| | | Glu35→GlN35 |
| HL 12B | 1A+2B+3B | Asp33→Lys33 |
| | | Glu35→GlN35 |
| | | Lys47→Arg47 |
| HL 14A | 1A+2C+3A | Asp33→Lys33 |
| | | Lys36→Arg36 |
| HL 14B | 1A+2C+3B | Asp33→Lys33 |
| | | Lys36→Arg36 |
| | | Lys47→Arg47 |
| HL 14C | 1A+2C+3C | Asp33→Lys33 |
| | | Lys36→Arg36 |
| | | Lys47→Gln47 |

Die nach der Ligation entstehenden chimären Plasmide (pRIT 2T-Hir) wurden zur DNA Amplifikation in einen Lambdalysogenen E. coli Stamm N 4830-1 (Pharmacia Bestell-Nr. 27-4808-01) transformiert, DNA aus Einzelklonen isoliert und durch DNA-Sequenzanalyse auf das Vorliegen der korrekten Sequenz hin überprüft.

### Beispiel 2

### Expression des Fusionsproteins

Das jeweilige Expressionsplasmid pRIT 2T-Hir wurde in den Stamm E.coli N 4830-1 transformiert. Dieser Stamm enthält chromosomal den thermosensitiven Lambda-Repressor CI 857.

In einem 1 1 Erlenmeyerkolben mit Schikanen wurden 100 ml MIM-Medium (MIM = 32 g Trypton, 20 g Hefeextrakt, 6 g Na₂HPO₄, 3 g KH₂PO₄, 0,5 g NaCl, 1 g NH₄Cl pro Liter und 0,1 mM mgSO₄ sowie 0,001 mM FeCl₃) sterilisiert und Ampicillin (ad 100 µg/ml) zugegeben. Das Medium wurde mit 1 ml einer frischen Über-Nacht-Kultur des Stammes pRIT 2TA-Hir/N 4830-1 angeimpft und unter Schütteln so lange bei 28°C bebrütet, bis die Absorption bei 550 nm 0,6 betrug. Dann wurden 100 ml 65°C warmes frisches MIM/amp-Medium zugegeben und weitere 4 h bei 42°C bebrütet. In dieser Zeit wurde das gewünschte Fusionsprotein synthetisiert. Durch Zugabe von Lysozym zu 75 mg/l und Inkubation (3h, 37°C) wurde die Zellwand enzymatisch entfernt. Die Zellen konnten dann mechanisch (Manton-Gaulin Presse, Einfrierzyklus, heftiges Rühren), durch einen Hitzeschock bis 80°C oder eine hypotone Lyse aufgeschlossen und das lösliche Fusionsprotein ins Medium freigesetzt werden.

### Beispiel 3

### Reinigung des Fusionsproteins

Die Zellbruchstücke wurden durch Zentrifugation entfernt und der klare Überstand über eine IgG-Sepharose-Säule (IgG-Sepharose® 6 "Fast Flow", Pharmacia, Best Nr. 17-0969-01) gepumpt. Die Lagerung des Säulenmaterials, Vorbereitung und Auslegung der Säule, Auftragsbedingungen und Flußraten richten sich nach den Angaben des Herstellers. So wurden für 6 1 Überstand ein Gelbett von 200 ml und eine Flußrate von ca. 3 1/h verwendet. Bei diesem Schritt wurde das Fusionsprotein über seinen IgG-bindenden Protein A-Teil reversibel an die Gelmatrix gebunden (Ausbeute ca. 95 %). Nach dem Auftrag wurde die Säule mit 10 Bettvolumina TST (50 mM Tris-HCl pH 7,6; 150 mM NaCl und 0,05 % Tween®20) gewaschen. Die Elution erfolgte mit 0,5 M Acetatpuffer pH 2,8.

### Beispiel 4

### Spaltung des Fusionsproteins

Das Säuleneluat aus Beispiel 3 wurde lyophilisiert und bei -20°C gelagert. Zur Spaltung wurde es in 70%iger Ameisensäure zu einer Proteinkonzentration von ca. 25 g/l aufgenommen. Nach Spülen mit Argon wurde zur Abspaltung des Hirudin-Muteins 1 g BrCN als Feststoff pro g Fusionsprotein zugegeben. Die Spaltung erfolgte unter Argon bei 37°C in ca. 4 h. Das überschüssige Bromycan, das Lösungsmittel und weitere flüchtige Bestandteile wurden durch Lyophilisation entfernt. Anschließend wurde dreimal mit Wasser gewaschen.

### Beispiel 5

### Renaturierung und Reinigung von Hirudin-Muteinen

Das Lyophilisat wurde zu 1-100 mg/ml Protein in 6 M Guanidin-HCl, 0,1 M Tris/HCl pH 8,5, 0,2 M DTT aufgenommen. Nach einer Inkubationszeit von 2 h wurde die Probe durch G-10-Ausschlußchromatographie (äquilibriert mit 10 mM HCl) entsalzt. Die entsalzte Probe wurde 1:20 in 0,1 M Tris/HCl, 5 mM GSH/0,5 mM GSSG, 1 mM EDTA, pH 8,7 verdünnt und 1 h inkubiert (GSH ist reduziertes und GSSH oxidiertes Glutathion). Durch diese Behandlung stieg die spezifische Aktivität des Hirudin-Muteins um den Faktor 3-5. Nach Einstellen des pH-Wertes auf pH 7,6 mit HCl, Zugabe von NaCl auf 150 mM und Tween®20 auf 0,05 % wurde die Chromatographie über IgG-Sepharose wiederholt (Beispiel 3). Während der Protein A-Fusionspartner und ungespaltenes Fusionsprotein an die Säule banden, befand sich das akt-ive Hirudin-Mutein mit einer Reinheit von > 90 % im Durchlauf. Es konnte durch klassiche proteinchemische Verfahren bis zur klinischen Reinheit weiter aufgereinigt werden.

### Beispiel 6

Darstellung des Methoxy-polyethylenglykol(8000)-N-succinimidocarbonats

### a) N-Succinimido-chloroformiat

In eine Lösung von ca. 30 g Phosgen in 200 ml Dichlormethan wurden bei 0°C innerhalb von 30 min 21,0 g N-Hydroxysuccinimid-Kaliumsalz eingetragen und das Gemisch 2 h bei 0°C gerührt. Anschließend wurde für 1 h Stickstoff durch die Suspension geleitet, um überschüssiges Phosgen abzublasen (Waschturm!). Die Suspension wurde filtriert und das Filtrat im Vacuum zur Trockene eingeengt. Die 10,6 g N-Succinimido-chloroformiat lagen als gelbliches Öl vor und waren mit anorganischen Salzen und Disuccinimido-carbonat verunreinigt. Das Rohprodukt konnte ohne weitere Reinigung für die Umsetzung mit Polyalkylenglykolen eingesetzt werden oder durch Lösen in 150 ml Diethylether, Filtration und erneutes Einengen von anorganischen Salzen befreit werden.

### b) Methoxy-polyethylenglykol(8000)-N-succinimido-carbonat

10,0 g Methoxy-PEG(8000)-OH wurden durch leichtes Erwärmen in 20 ml trockenem Pyridin gelöst. Nach dem Abkühlen auf Raumtemperatur wurde die Lösung mit 890 mg N-Succinimido-chloroformiat versetzt und über Nacht gerührt. Nach Zugabe von einem Überschuß Diethylether wurde das Gemisch 30 min im Eisbad gerührt, der ausgefallene Feststoff abfiltriert, 2 mal aus Isopropanol umkristallisiert, aus Diethylether gefällt, filtriert und getrocknet. 8,10 g Methoxy-polyethylenglykol(8000)-N-succinimidocarbonat fielen als farbloser Feststoff an.

### Beispiel 7

Darstellung von Methoxy-polyethylenglykol(8000)-4-nitrophenyl-carbonat

### a) 4-Nitrophenyl-chloroformiat

In eine Suspension von 20,0 g Nitrophenol in 60 ml Toluol wurden bei 0°C ca. 43 g Phosgen eingegast. Das Gemisch wurde 4-5 h bei 0°C gerührt. Anschließend wurde bei -15°C langsam eine Lösung von 20 ml Triethylamin in 20 ml Toluol zugetropft und über Nacht im auftauenden Kältebad gerührt. Überschüssiges Phosgen wurde durch Stickstoff ausgeblasen und das Reaktionsgemisch anschließend filtriert. Das Filtrat wurde im Vakuum bis zur Trockene eingeengt. Die 33,7 g öliger, bräunlicher Rückstand enthielten neben 4-Nitrophenol-chloroformiat noch Lösungsmittel und Salz. Das Gemisch kristallisierte im Kühlschrank und konnte ohne weitere Reinigung eingesetzt werden.

### b) Methoxy-polyethylenglykol(8000)-4-nitrophenyl-carbonat

Die Darstellung und Reinigung erfolgte analog Beispiel 6.

### Beispiel 8

Darstellung von Methoxy-polyethylenglykol(8000)-2,4,5-trichlorphenylcarbonat

### a) 2,4,5-Trichlorphenyl-chloroformiat

In eine Lösung von 10,0 g 2,4,5-Trichlorphenol in 50 ml Dichlormethan wurden bei 0°C ca. 7 g Phosgen eingegast und das Gemisch 15 min bei 0°C gerührt. 7,2 ml Chinolin in 20 ml Dichlormethan wurden innerhalb von 30 min zugetropft und die orangefarbene Suspension noch 1 h im Eisbad gerührt. Anschließend wurde 1 h Stickstoff durch die Suspension geleitet, um überschüssiges Phosgen abzublasen (Waschturm). Das Gemisch wurde anschließend filtriert, das Filtrat 2 mal mit Wasser gewaschen, getrocknet, erneut filtriert und im Vakuum bis zur Trockene eingeengt. Die 4,45 g öliger, bräunlicher Rückstand sind relativ sauberes 2,4,5-Trichlorphenylchloroformiat, das im Kühlschrank kristallisiert und ohne weitere Reinigung eingesetzt werden kann.

### b) Methoxy-polyethylenglykol(8000)-2,4,5-trichlor-phenylcarbonat

Die Darstellung und Reinigung erfolgte analog Beispiel 6.

### Beispiel 9

Umsetzung des Hirudin-Muteins HL 14B mit Methoxy-polyethylen-glykol(8000)-4-nitrophenylcarbonat

10 mg des Hirudin-Muteins HL 14B (spezifische Aktivität 8900 ATU/mg) wurden zu einer Konzentration von 20 mg/ml in 0,1 M Natriumcarbonat-Puffer pH 8,0 gelöst, mit 80 mg 4-Nitrophenyl-aktiviertem Methoxypolyethylenglykol (8000 Da), gelöst in 0,5 ml 1,4-Dioxan, versetzt und 3 h bei 25°C inkubiert. Die Reaktion wurde dann durch Zusatz eines 100fachen Überschusses an Tris-Base abgestoppt, freigesetztes 4-Nitrophenol durch Extraktion entfernt und das Hirudin-PEG Konjugat auf eine HP-Q-Sepharose®-Säule (Pharmacia®) aufgetragen. Die Säule wurde mit einem linearen NaCl-Gradienten von 0 bis 400 mM NaCl in 20 mM Tris/HCl, pH 8,0 entwickelt.

Der Anteil des gewünschten PEG2-Derivates im Kopplungsansatz lag bei ca. 80-85 %; der Anteil der ungewünschten PEG₁- und PEG₃-Derivate betrug jeweils maximal etwa 5-10 %. Die spezifische Aktivität des gereinigten PEG₂-HL 14B-Konjugates betrug 8300 U/mg Protein. Das Molgewicht wurde durch Superose®-Gelfiltration zu 22000-23000 Da bestimmt.

## Patentansprüche

1. Konjugate der Formel I aus Polyalkylenglykol oder Polyalkylenglykolderivaten mit blutgerinnungshemmenden Hirudin-Muteinen
[Z-(CH₂)ₙ-[O-(CH₂)ₙ]ₘ-W-]ₚHir I
worin
Z einen der Reste -OH, -NH2, -NH-CO-R, -O-R oder -O-CO-R (mit R in der Bedeutung einer C₁-C₄-Alkylgruppe),
n die Zahl 2, 3 oder 4,
m eine Zahl von 50 bis 500,
W eine direkte kovalente Bindung oder einen Linker,
p die Zahl 1, 2 oder 3
bedeuten und
Hir für ein über die Aminogruppe(n) der Lysinseitenkette(n) an den (die) Z-(CH₂)ₙ-[O-(CH₂)ₙ]ₘ-W-Rest (e) gebundenes Polypeptid der Formel II 
A -Tyr-Thr-Asp-Cys-Thr-Glu-Ser-Gly-
10
Gln-Asn-Leu-Cys-Leu-Cys-Glu-Gly-Ser-Asn-
20
Val-Cys-Gly- B -Gly-Asn- C -Cys-Ile-Leu-
30 II
Gly-Ser-Asp-Gly-Glu-Lys-Asn-Gln-Cys-Val-
40
Thr-Gly-Glu-Gly-Thr-Pro- D -Pro-Gln-Ser-
50
His-Asn-Asp-Gly-Asp-Phe-Glu- E -Ile-Pro-
60
Glu-Glu-Tyr-Leu- F
65
 steht, mit der Bedeutung von
A = Val-Val,
= Ile-Thr,
= Leu-Thr oder
= Pro-Val,
B = Gln oder Glu,
C = Lys, Arg oder Asn,
D = Lys, Arg, Asn oder Gln,
E = Glu oder Pro,
F = Asp oder Gln,
wobei im Polypeptid II ein oder mehrere Aminosäuren in den Positionen 30-38 ausgetauscht oder deletiert und ein oder zwei zusätzliche Lysinreste durch Substitution einer vorhandenen Aminosäure in den Positionen 30 bis 38 enthalten sind.

2. Konjugate gemäß Anspruch 1, dadurch gekennzeichnet, daß das Polyalkylenglykol ein Polyethylenglykol ist.

3. Konjugate gemäß Anspruch 1, dadurch gekennzeichnet, daß das Molgewicht des Polyethylenglykols zwischen 4.000 und 15.000 Da liegt.

4. Konjugate gemäß den Ansprüchen 1 bis 3, dadurch gekennzeichnet, daß ein oder zwei Polymerreste am Hirudinmutein angeknüpft sind.

5. Konjugate gemäß den Ansprüchen 1 bis 4, dadurch gekennzeichnet, daß D Arg oder Gln ist.

6. Konjugat der Formel I gemäß Anspruch 1, dadurch gekennzeichnet, daß Hir für ein Polypeptid der Formel 
Val-Val-Tyr-Thr-Asp-Cys-Thr-Glu-Ser-Gly-
10
Gln-Asn-Leu-Cys-Leu-Cys-Glu-Gly-Ser-Asn-
20
Val-Cys-Gly-Gln-Gly-Asn-Lys-Cys-Ile-Leu-
30
Gly-Ser-Lys-Gly-Glu-Arg-Asn-Gln-Cys-Val-
40
Thr-Gly-Glu-Gly-Thr-Pro-Arg-Pro-Gln-Ser-
50
His-Asn-Asp-Gly-Asp-Phe-Glu-Glu-Ile-Pro-
60
Glu-Glu-Tyr-Leu-Gln
65
 steht.

7. Konjugate gemäß den Ansprüchen 1 bis 6 zur Bekämpfung von Krankheiten.

8. Konjugate gemäß den Ansprüchen 1 bis 6 zur Beschichtung von Oberflächen.

9. Hirudinmuteine der Formel H-Hir, worin Hir die in Anspruch 1 angegebene Bedeutung besitzt.

10. Hirudinmutein gemäß Anspruch 9, dadurch gekennzeichnet, daß D Arg oder Gln ist.

11. Hirudinmutein gemäß Anspruch 9 der Formel

Val-Val-Tyr-Thr-Asp-Cys-Thr-Glu-Ser-Gly-
10
Gln-Asn-Leu-Cys-Leu-Cys-Glu-Gly-Ser-Asn-
20
Val-Cys-Gly-Gln-Gly-Asn-Lys-Cys-Ile-Leu-
30
Gly-Lys-Asn-Gly-Gln-Lys-Asn-Gln-Cys-Val-
40
Thr-Gly-Glu-Gly-Thr-Pro-Lys-Pro-Gln-Ser-
50
His-Asn-Asp-Gly-Asp-Phe-Glu-Glu-Ile-Pro-
60
Glu-Glu-Tyr-Leu-Gln.
65

12. Hirudinmutein gemäß Anspruch 9 der Formel

Val-Val-Tyr-Thr-Asp-Cys-Thr-Glu-Ser-Gly-
10
Gln-Asn-Leu-Cys-Leu-Cys-Glu-Gly-Ser-Asn-
20
Val-Cys-Gly-Gln-Gly-Asn-Lys-Cys-Ile-Leu-
30
Gly-Ser-Lys-Gly-Gln-Lys-Asn-Gln-Cys-Val-
40
Thr-Gly-Glu-Gly-Thr-Pro-Lys-Pro-Gln-Ser-
50
His-Asn-Asp-Gly-Asp-Phe-Glu-Glu-Ile-Pro-
60
Glu-Glu-Tyr-Leu-Gln.
65

13. Hirudinmutein gemäß Anspruch 9 der Formel

Val-Val-Tyr-Thr-Asp-Cys-Thr-Glu-Ser-Gly-
10
Gln-Asn-Leu-Cys-Leu-Cys-Glu-Gly-Ser-Asn-
20
Val-Cys-Gly-Gln-Gly-Asn-Lys-Cys-Ile-Leu-
30
Gly-Ser-Lys-Gly-Glu-Arg-Asn-Gln-Cys-Val-
40
Thr-Gly-Glu-Gly-Thr-Pro-Lys-Pro-Gln-Ser-
50
His-Asn-Asp-Gly-Asp-Phe-Glu-Glu-Ile-Pro-
60
Glu-Glu-Tyr-Leu-Gln.
65

14. Hirudinmutein gemäß Anspruch 9 der Formel

Val-Val-Tyr-Thr-Asp-Cys-Thr-Glu-Ser-Gly
10
Gln-Asn-Leu-Cys-Leu-Cys-Glu-Gly-Ser-Asn-
20
Val-Cys-Gly-Gln-Gly-Asn-Lys-Cys-Ile-Leu-
30
Gly-Ser-Lys-Gly-Glu-Arg-Asn-Gln-Cys-Val-
40
Thr-Gly-Glu-Gly-Thr-Pro-Arg-Pro-Gln-Ser-
50
His-Asn-Asp-Gly-Asp-Phe-Glu-Glu-Ile-Pro-
60
Glu-Glu-Tyr-Leu-Gln.
65

15. Hirudinmutein gemäß Anspruch 9 der Formel

Val-Val-Tyr-Thr-Asp-Cys-Thr-Glu-Ser-Gly-
10
Gln-Asn-Leu-Cys-Leu-Cys-Glu-Gly-Ser-Asn-
20
Val-Cys-Gly-Gln-Gly-Asn-Lys-Cys-Ile-Leu-
30
Gly-Ser-Lys-Gly-Glu-Arg-Asn-Gln-Cys-Val-
40
Thr-Gly-Glu-Gly-Thr-Pro-Gln-Pro-Gln-Ser
50
His-Asn-Asp-Gly-Asp-Phe-Glu-Glu-Ile-Pro-
60
Glu-Glu-Tyr-Leu-Gln.
65

16. Hirudinmuteine gemäß den Ansprüchen 9 bis 15 zur Beschichtung von Oberflächen.

## Claims

1. A conjugate of the formula I of polyalkylene glycol or polyalkylene glycol derivatives with anticoagulant hirudin muteins
[Z-(CH₂)ₙ-[O-(CH₂)ₙ]ₘ-W-]_{P}Hir I
in which
Z denotes one of the radicals -OH, -NH₂, -NH-CO-R, -O-R or -O-CO-R (with R meaning a C₁-C₄-alkyl group),
n denotes the number 2, 3 or 4,
m denotes a number from 50 to 500,
W denotes a direct covalent bond or a linker,
p denotes the number 1, 2 or 3 and
Hir represents a polypeptide of the formula II 
A -Tyr-Thr-Asp-Cys-Thr-Glu-Ser-Gly-
10
Gln-Asn-Leu-Cys-Leu-Cys-Glu-Gly-Ser-Asn-
20
Val-Cys-Gly- B -Gly-Asn- C -Cys-Ile-Leu-
-
30
Gly-Ser-Asp-Gly-Glu-Lys-Asn-Gln-Cys-Val-
40
Thr-Gly-Glu-Gly-Thr-Pro- D -Pro-Gln-Ser-
50
His-Asn-Asp-Gly-Asp-Phe-Glu- E -Ile-Pro-
60
Glu-Glu-Tyr-Leu- F
65
which is bonded via the amino group(s) of the lysine side-chain(s) to the Z-(CH₂)ₙ-[O-(CH₂)ₙ]ₘ-W radical(s),
with the meaning of
A = Val-Val,
= Ile-Thr,
= Leu-Thr or
= Pro-Val,
B = Gln or Glu,
C = Lys, Arg or Asn,
D = Lys, Arg, Asn or Gln,
E = Glu or Pro,
F = Asp or Gln,
where in the polypeptide II one or more amino acids in positions 30-38 have been replaced or deleted and one or two additional lysine residues are present due to replacement of an existing amino acid in positions 30 to 38.

2. A conjugate as claimed in claim 1, wherein the polyalkylene glycol is a polyethylene glycol.

3. A conjugate as claimed in claim 1, wherein the molecular weight of the polyethylene glycol is between 4,000 and 15,000 Da.

4. A conjugate as claimed in any of claims 1 to 3, wherein one or two polymer residues are linked to the hirudin mutein.

5. A conjugate as claimed in any of claims 1 to 4, wherein D is Arg or Gln.

6. A conjugate of the formula I as claimed in claim 1, wherein Hir is a polypeptide of the formula

Val-Val-Tyr-Thr-Asp-Cys-Thr-Glu-Ser-Gly-
10
Gln-Asn-Leu-Cys-Leu-Cys-Glu-Gly-Ser-Asn-
20
Val-Cys-Gly-Gln-Gly-Asn-Lys-Cys-Ile-Leu-
30
Gly-Ser-Lys-Gly-Glu-Arg-Asn-Gln-Cys-Val-
40
Thr-Gly-Glu-Gly-Thr-Pro-Arg-Pro-Gln-Ser-
50
His-Asn-Asp-Gly-Asp-Phe-Glu-Glu-Ile-Pro-
60
Glu-Glu-Tyr-Leu-Gln
65

7. A conjugate as claimed in any of claims 1 to 6 for controlling diseases.

8. A conjugate as claimed in any of claims 1 to 6 for coating surfaces.

9. A hirudin mutein of the formula H-Hir, in which Hir has the meaning indicated in claim 1.

10. A hirudin mutein as claimed in claim 9, wherein D is Arg or Gln.

11. A hirudin mutein as claimed in claim 9 of the formula

Val-Val-Tyr-Thr-Asp-Cys-Thr-Glu-Ser-Gly-
10
Gln-Asn-Leu-Cys-Leu-Cys-Glu-Gly-Ser-Asn-
20
Val-Cys-Gly-Gln-Gly-Asn-Lys-Cys-Ile-Leu-
30
Gly-Lys-Asn-Gly-Gln-Lys-Asn-Gln-Cys-Val-
40
Thr-Gly-Glu-Gly-Thr-Pro-Lys-Pro-Gln-Ser-
50
His-Asn-Asp-Gly-Asp-Phe-Glu-Glu-Ile-Pro-
60
Glu-Glu-Tyr-Leu-Gln.
65

12. A hirudin mutein as claimed in claim 9 of the formula

Val-Val-Tyr-Thr-Asp-Cys-Thr-Glu-Ser-Gly-
10
Gln-Asn-Leu-Cys-Leu-Cys-Glu-Gly-Ser-Asn-
20
Val-Cys-Gly-Gln-Gly-Asn-Lys-Cys-Ile-Leu-
30
Gly-Ser-Lys-Gly-Gln-Lys-Asn-Gln-Cys-Val-
40
Thr-Gly-Glu-Gly-Thr-Pro-Lys-Pro-Gln-Ser-
50
His-Asn-Asp-Gly-Asp-Phe-Glu-Glu-Ile-Pro-
60
Glu-Glu-Tyr-Leu-Gln.
65

13. A hirudin mutein as claimed in claim 9 of the formula

Val-Val-Tyr-Thr-Asp-Cys-Thr-Glu-Ser-Gly-
10
Gln-Asn-Leu-Cys-Leu-Cys-Glu-Gly-Ser-Asn-
20
Val-Cys-Gly-Gln-Gly-Asn-Lys-Cys-Ile-Leu-
30
Gly-Ser-Lys-Gly-Glu-Arg-Asn-Gln-Cys-Val-
40
Thr-Gly-Glu-Gly-Thr-Pro-Lys-Pro-Gln-Ser-
50
His-Asn-Asp-Gly-Asp-Phe-Glu-Glu-Ile-Pro-
60
Glu-Glu-Tyr-Leu-Gln.
65

14. A hirudin mutein as claimed in claim 9 of the formula

Val-Val-Tyr-Thr-Asp-Cys-Thr-Glu-Ser-Gly
10
Gln-Asn-Leu-Cys-Leu-Cys-Glu-Gly-Ser-Asn-
20
Val-Cys-Gly-Gln-Gly-Asn-Lys-Cys-Ile-Leu-
30
Gly-Ser-Lys-Gly-Glu-Arg-Asn-Gln-Cys-Val-
40
Thr-Gly-Glu-Gly-Thr-Pro-Arg-Pro-Gln-Ser-
50
His-Asn-Asp-Gly-Asp-Phe-Glu-Glu-Ile-Pro-
60
Glu-Glu-Tyr-Leu-Gln.
65

15. A hirudin mutein as claimed in claim 9 of the formula

Val-Val-Tyr-Thr-Asp-Cys-Thr-Glu-Ser-Gly-
10
Gln-Asn-Leu-Cys-Leu-Cys-Glu-Gly-Ser-Asn-
20
Val-Cys-Gly-Gln-Gly-Asn-Lys-Cys-Ile-Leu-
30
Gly-Ser-Lys-Gly-Glu-Arg-Asn-Gln-Cys-Val-
40
Thr-Gly-Glu-Gly-Thr-Pro-Gln-Pro-Gln-Ser
50
His-Asn-Asp-Gly-Asp-Phe-Glu-Glu-Ile-Pro-
60
Glu-Glu-Tyr-Leu-Gln.
65

16. A hirudin mutein as claimed in any of claims 9 to 15 for coating surfaces.

## Revendications

1. Conjugués de formule I de polyalkylèneglycol ou dérivés de polyalkylèneglycol avec des mutéines d'hirudine entravant la coagulation du sang
[Z-(CH₂)n-[O-(CH₂)ₙ]ₘ-W-]ₚHir I
où
Z représente un des restes -OH, -NH₂, -NH-CO-R, -O-R ou -O-CO-R (R étant mis pour un groupe alkyle en C1-C4),
n représente un des nombres 2, 3 ou 4,
m représente un nombre compris entre 50 et 500,
W est une liaison covalente directe ou un linker,
p est un des nombres 1, 2 ou 3
et
Hir est mis pour un polypeptide de formule II lié au (aux) reste (restes) Z-(CH₂)ₙ-[O-(CH₂)ₙ]ₘ-W par l'intermédiaire du (des) groupe (s) amino de la (des) chaîne(s) de lysine latérale(s), 
A -Tyr-Thr-Asp-Cys-Thr-Glu-Ser-Gly-
10
Gln=Asn-Leu-Cys-Leu-Cys-Glu-Gly-Ser-Asn-
20
Val-Cys-Gly- B -Gly-Asn- C -Cys-Ile-Leu-
-
30
II
Gly-Ser-Asp-Gly-Glu-Lys-Asn-Gln-Cys-Val-
40
Thr-Gly-Glu-Gly-Thr-Pro- D -Pro-Gln-Ser-
50
His-Asn-Asp-Gly-Asp-Phe-Glu- E -Ile-Pro-
60
Glu-Glu-Tyr-Leu- F
65
 aux significations
A = Val-Val,
= Ile-Thr,
= Leu-Thr ou
= Pro-Val,
B = Gln ou Glu,
C = Lys, Arg ou Asn,
D = Lys, Arg, Asn ou Gln,
E = Glu ou Pro,
F = Asp ou Gln,
un ou plusieurs amino-acides dans le polypeptide II étant échangés ou éliminés par délétion dans les positions 30 à 38 et un ou deux restes lysine additionnels étant contenus dans les positions 30 à 38 par substitution d'un amino-acide présent.

2. Conjugués selon la revendication 1, caractérisés par le fait que le polyalkylèneglycol est un polyéthylèneglycol.

3. Conjugués selon la revendication 1, caractérisés par le fait que le poids moléculaire du polyéthylèneglycol varie entre 4.000 et 15.000 Da.

4. Conjugués selon les revendications 1 à 3, caractérisés par le fait qu'un ou deux restes polymères sont accolés sur la mutéine d'hirudine.

5. Conjugués selon les revendications 1 à 4, caractérisés par le fait que D est Arg ou Gln.

6. Conjugués de formule I, selon la revendication 1, caractérisés par le fait que Hir est mis pour un polypeptide de formule

Val-Val-Tyr-Thr-Asp-Cys-Thr-Glu-Ser-Gly-
10
Gln-Asn-Leu-Cys-Leu-Cys-Glu-Gly-Ser-Asn-
20
Val-Cys-Gly-Gln-Gly-Asn-Lys-Cys-Ile-Leu-
30
Gly-Ser-Lys-Gly-Glu-Arg-Asn-Gln-Cys-Val-
40
Thr-Gly-Glu-Gly-Thr-Pro-Arg-Pro-Gln-Ser-
50
His-Asn-Asp-Gly-Asp-Phe-Glu-Glu-Ile-Pro-
60
Glu-Glu-Tyr-Leu-Gln
65

7. Conjugués selon les revendications 1 à 6 pour la lutte contre certaines maladies.

8. Conjugués selon les revendications 1 à 6 pour l'enduction de surfaces.

9. Mutéines d'hirudine de formule H-Hir, dans laquelle Hir a la signification donnée dans la revendication 1.

10. Mutéine d'hirudine selon la revendication 9, caractérisé par le fait que D est Arg ou Gln.

11. Mutéine d'hirudine selon la revendication 9, de formule

Val-Val-Tyr-Thr-Asp-Cys-Thr-Glu-Ser-Gly-
10
Gln-Asn-Leu-Cys-Leu-Cys-Glu-Gly-Ser-Asn-
20
Val-Cys-Gly-Gln-Gly-Asn-Lys-Cys-Ile-Leu-
30
Gly-Lys-Asn-Gly-Gln-Lys-Asn-Gln-Cys-Val-
40
Thr-Gly-Glu-Gly-Thr-Pro-Lys-Pro-Gln-Ser-
50
His-Asn-Asp-Gly-Asp-Phe-Glu-Glu-Ile-Pro-
60
Glu-Glu-Tyr-Leu-Gln.
65

12. Mutéine d'hirudine selon la revendication 9, de formule

Val-Val-Tyr-Thr-Asp-Cys-Thr-Glu-Ser-Gly-
10
Gln-Asn-Leu-Cys-Leu-Cys-Glu-Gly-Ser-Asn-
20
Val-Cys-Gly-Gln-Gly-Asn-Lys-Cys-Ile-Leu-
30
Gly-Ser-Lys-Gly-Gln-Lys-Asn-Gln-Cys-Val-
40
Thr-Gly-Glu-Gly-Thr-Pro-Lys-Pro-Gln-Ser-
50
His-Asn-Asp-Gly-Asp-Phe-Glu-Glu-Ile-Pro-
60
Glu-Glu-Tyr-Leu-Gln.
65

13. Mutéine d'hirudine selon la revendication 9, de formule

Val-Val-Tyr-Thr-Asp-Cys-Thr-Glu-Ser-Gly-
10
Gln-Asn-Leu-Cys-Leu-Cys-Glu-Gly-Ser-Asn-
20
Val-Cys-Gly-Gln-Gly-Asn-Lys-Cys-Ile-Leu-
30
Gly-Ser-Lys-Gly-Glu-Arg-Asn-Gln-Cys-Val-
40
Thr-Gly-Glu-Gly-Thr-Pro-Lys-Pro-Gln-Ser-
50
His-Asn-Asp-Gly-Asp-Phe-Glu-Glu-Ile-Pro-
60
Glu-Glu-Tyr-Leu-Gln.
65

14. Mutéine d'hirudine selon la revendication 9, de formule

Val-Val-Tyr-Thr-Asp-Cys-Thr-Glu-Ser-Gly
10
Gln-Asn-Leu-Cys-Leu-Cys-Glu-Gly-Ser-Asn-
20
Val-Cys-Gly-Gln-Gly-Asn-Lys-Cys-Ile-Leu-
30
Gly-Ser-Lys-Gly-Glu-Arg-Asn-Gln-Cys-Val-
40
Thr-Gly-Glu-Gly-Thr-Pro-Arg-Pro-Gln-Ser-
50
His-Asn-Asp-Gly-Asp-Phe-Glu-Glu-Ile-Pro-
60
Glu-Glu-Tyr-Leu-Gln.
65

15. Mutéine d'hirudine selon la revendication 9, de formule

Val-Val-Tyr-Thr-Asp-Cys-Thr-Glu-Ser-Gly-
10
Gln-Asn-Leu-Cys-Leu-Cys-Glu-Gly-Ser-Asn-
20
Val-Cys-Gly-Gln-Gly-Asn-Lys-Cys-Ile-Leu-
30
Gly-Ser-Lys-Gly-Glu-Arg-Asn-Gln-Cys-Val-
40
Thr-Gly-Glu-Gly-Thr-Pro-Gln-Pro-Gln-Ser
50
His-Asn-Asp-Gly-Asp-Phe-Glu-Glu-Ile-Pro-
60
Glu-Glu-Tyr-Leu-Gln.
65

16. Mutéines d'hirudine selon les revendications 9 à 15 pour l'enduction de surfaces.
